(19) Europäisches Patentamt
European Patent Office
Office européen des brevets

(11) Numéro de publication : **0 479 644 A1**

(12) # DEMANDE DE BREVET EUROPEEN

(21) Numéro de dépôt : **91402533.3**

(22) Date de dépôt : **24.09.91**

(51) Int. Cl.[5] : **B01J 27/051,** B01J 27/51, C07C 17/152, C01G 39/00

(30) Priorité : **02.10.90 FR 9012116**

(43) Date de publication de la demande : **08.04.92 Bulletin 92/15**

(84) Etats contractants désignés : **AT BE CH DE DK ES FR GB GR IT LI LU NL SE**

(71) Demandeur : **ATOCHEM**
**4 & 8, Cours Michelet La Défense 10**
**F-92800 Puteaux (FR)**

(72) Inventeur : **Lesparre, Jean**
**7 Le Coût**
**F-38560 Champ Sur Drac (FR)**
Inventeur : **Sergent, Marcel**
**21, rue des Tilleuls**
**F-35510 Cesson-Sevigne (FR)**
Inventeur : **Chevrel, Roger**
**Le Pont Vignoc**
**F-35630 Hede (FR)**
Inventeur : **Rabiller-Baudry, Murielle**
**146, Rue de Fougères**
**F-35700 Rennes (FR)**
Inventeur : **Faure, Annick**
**18 , rue Ludovic Bonin**
**F-69200 Venissieux (FR)**

(54) **Catalyseurs d'oxychloration a base de chalcogenures ternaires de molybdene.**

(57) La présente invention concerne des chalcogénures de formule $M_xMo_6Ch_8$ dans laquelle M désigne un métal, Ch désigne le soufre, le selenium ou le tellure et x est compris entre 0 et 4.
Ces chalcogénures sont utiles comme catalyseur d'oxychloration.

EP 0 479 644 A1

Jouve, 18, rue Saint-Denis, 75001 PARIS

La présente invention concerne des catalyseurs d'oxychloration à base de chalcogénures ternaires de molybdène. Le 1,2-dichloroéthane (D 12) est un produit fabriqué industriellement à raison de plusieurs millions de tonnes par an, qui par pyrolyse donne du chlorure de vinyle monomère (VCM) et de l'acide chlorhydrique (HCl). Le VCM est polymérisé en poly(chlorure de vinyle) (ou PVC) matière plastique couramment utilisée. L'HCl obtenu à la pyrolyse est séparé du VCM puis est mis en contact avec de l'éthylène et de l'oxygène en présence d'un catalyseur pour donner du D 12, c'est la réaction d'oxychloration. Cette réaction est très générale et peut être effectuée avec la plupart des hydrocarbures. L'oxychloration a été décrite dans de nombreux brevets, en particulier dans FR 2 063 365, FR 2 260 551, FR 2 242 143, FR 2 213 259 et FR 2 125 748. Le catalyseur est constitué d'un sel de cuivre déposé sur de l'alumine en poudre.

On a maintenant trouvé un nouveau procédé d'oxychloration dans lequel le catalyseur est un chalcogénure ternaire de molybdène contenant du cuivre.

La présente invention est donc un procédé d'oxychloration d'un hydrocarbure pour former un hydrocarbure chloré dans lequel l'hydrocarbure, un gaz contenant de l'oxygène et de l'acide chlorhydrique gazeux passent sur une charge comprenant un chalcogénure ternaire de molybdène contenant du cuivre et de formule:

$$Cu_x\ Mo_6\ Ch_8$$

dans laquelle x est un nombre quelconque entre 0 et 4 et Ch représente le soufre, le selenium ou le tellure.

Ces chalcogénures peuvent être des composés pseudo-moléculaires basés sur le motif $Mo_6\ Ch_8$ qui constitue un réseau hôte à la structure rigide, développant des canaux sécants dans les trois dimensions où viennent se loger les cations Cu.

Ces phases pseudo-moléculaires peuvent également être présentées comme plusieurs réseaux imbriqués : un réseau d'atomes métalliques (l'octaèdre $Mo_6$) inscrit dans un réseau pseudo-cubique anionique de chalcogène ("cube" $Ch_8$), enfin un réseau de tunnels qui se développent entre les motifs $Mo_6\ Ch_8$. Ces tunnels sont ensuite occupés par les cations Cu qui stabilisent la structure. Ces chalcogénures cristallisent dans le système hexagonal-rhomboedrique.

Ces chalcogénures s'utilisent habituellement sous forme de poudres ou de granulés.

L'hydrocarbure peut être un mélange de plusieurs hydrocarbures, choisis parmi les hydrocarbures aliphatiques en $C_1$ à $C_{20}$, les cycloaliphatiques jusqu'en $C_{12}$ et les aromatiques ayant jusqu'à quatre noyaux benzéniques condensés ainsi que leurs dérivés de substitution chlorés. Ils peuvent être choisis par exemple parmi le méthane, l'éthane, le propane, l'éthylène et le propylène. L'invention est particulièrement utile pour l'éthylène. Le gaz contenant de l'oxygène est tout simplement de l'air mais on peut aussi l'utiliser, appauvri ou enrichi en oxygène.

Le but de l'oxychloration est essentiellement d'utiliser l'acide chlorhydrique comme source de chlore. On ajuste donc la quantité d'oxygène et d'hydrocarbure pour obtenir à peu près stoéchiométriquement l'hydrocarbure chloré en consommant le maximum d'HCl et d'hydrocarbure.

Dans le cas de l'éthylène la réaction est :

$$C_2H_4 + \frac{1}{2} O_2 + 2\ HCl \rightarrow C_2H_4\ Cl_2\ (D\ 12) + H_2O$$

bien qu'on puisse opérer en lit fixe ou en lit fluidisé, on préfère le lit fluidisé.

Lorsque l'on opère avec un catalyseur disposé en lit fluidisé, la température à laquelle s'effectue la réaction d'oxychloration se situe habituellement entre 150 et 450°C. De préférence, cette température est comprise entre 200 et 300°C.

La pression à laquelle est effectuée la réaction d'oxychloration n'est pas critique en elle-même. Habituellement, on opère avec des pressions comprises entre 1 et 10 atm et de préférence avec des pressions comprises entre 1 et 8 atm.

La vitesse de fluidisation des compositions catalytiques n'est pas critique en elle-même et dépend essentiellement de la granulométrie du catalyseur et des dimensions de l'appareillage. Généralement, on opère avec des vitesses comprises entre 5 et 100 cm/s et de préférence entre 10 et 50 cm/s.

Enfin, le rapport des réactifs mis en oeuvre est le même que celui généralement utilisé dans les procédés antérieurs. D'habitude, on opère avec un léger excès d'éthylène par rapport à la quantité d'HCl mis en oeuvre. Toutefois, les compositions catalytiques de l'invention permettent également de travailler au voisinage de la stoéchiométrie, voire même en excès d'HCl.

Toutes ces conditions opératoires sont connues de l'art antérieur.

L'avantage de l'invention est que le catalyseur est beaucoup plus résistant mécaniquement que les catalyseurs d'oxychloration de l'art antérieur, toutefois les performances en conversion de l'hydrocarbure de l'acide chlorhydrique et sélectivité en dichloroéthane ne sont pas bonnes.

La demanderesse a découvert que si on ajoute à ces chalcogénures des catalyseurs connus d'oxychloration, on obtient des performances supérieures à celles des catalyseurs connus. Ces catalyseurs connus désignent les catalyseurs conventionnels constitués de cuivre déposé sur un support poreux pouvant être de

l'alumine, c'est-à-dire qu'on opère avec un mélange de chalcogénure et de catalyseurs conventionnels. Ce sont par exemple les catalyseurs décrits dans les brevets FR 2 125 748, FR 2 063 365, FR 2 141 452, EP 57796, EP 62320, EP 119933 et EP 255156.

Ces catalyseurs connus sont avantageusement des poudres essentiellement à base d'alumine de granulométrie entre 20 et 200 μ et de surface entre 90 et 450 $m^2/g$ et de préférence entre 30 et 90 μ et entre 250 et 400 $m^2/g$. Ces poudres sont imprégnées de cuivre ou d'un sel de cuivre en quantité pouvant aller jusqu'à 10 %, et de préférence 3 à 10 % en poids du cuivre par rapport au catalyseur fini.

La demanderesse a aussi découvert qu'on pouvait ajouter à ces chalcogénures des catalyseurs d'oxychloration qui sont eux-mêmes constitués d'un mélange d'un catalyseur d'oxychloration et d'une substance solide catalytiquement et chimiquement inerte. C'est-à-dire qu'on opère avec un mélange d'un chalcogénure, d'un catalyseur conventionnel d'oxychloration et d'une substance inerte. Des catalyseurs d'oxychloration constitués d'un mélange de catalyseur et d'une substance inerte sont décrits dans le brevet FR 2 242 143.

A titre d'exemple comme substance catalytiquement et chimiquement inerte on peut citer les micro-billes de verre ou de silice, l'alumine alpha et, de préférence, le sable siliceux que l'on trouve à l'état naturel et dont la répartition granulométrique de la taille des particules est adaptée aux besoins du lit fixe ou du lit fluide.

Avantageusement la taille des particules inertes est comprise entre 20 et 200 μ.

La quantité de substance inerte peut varier dans de larges limites. Avantageusement la quantité d'inerte peut représenter de 1 à 20 fois en poids la quantité de catalyseur, c'est-à-dire de l'ensemble chalcogénure et catalyseur conventionnel d'oxychloration.

La demanderesse a aussi découvert qu'on pouvait ajouter à ces chalcogénures du support de catalyseur non imprégné, c'est-à-dire des poudres à base d'alumine ne contenant pas de cuivre. Ce sont par exemple les poudres de granulométrie entre 20 et 200 μ et de surface entre 90 et 450 $m^2/g$ qu'on a citées précédemment mais non imprégnées de cuivre.

On peut aussi ajouter en plus du support de catalyseur non imprégné une substance solide catalytiquement et chimiquement inerte. Cette substance a été définie précédemment : micro-billes de verre ou de silice, alumine alpha ...

Le procédé d'oxychloration de l'invention peut donc être mis en oeuvre avec les charges catalytiques suivantes :

- les chalcogénures,
- un mélange de chalcogénures et de catalyseurs conventionnels d'oxychloration et éventuellement une substance catalytiquement et chimiquement inerte,
- un mélange de chalcogénures et de support de catalyseur non imprégné et éventuellement une substance catalytiquement et chimiquement inerte.

Ces charges catalytiques peuvent être utilisées en lit fixe ou en lit fluidisé. Les chalcogénures précédents, c'est-à-dire les chalcogénures $Cu_x$ $Mo_6$ $Ch_8$, sont connus (J. Solid State Chem. 3, 515, 519 (1971) par contre les charges catalytiques comprenant un mélange de chalcogénures et de catalyseurs conventionnels d'oxychloration et éventuellement une substance catalytiquement et chimiquement inerte sont nouvelles.

De même pour les mélanges de chalcogénures et de support de catalyseur non imprégné et éventuellement une substance catalytiquement et chimiquement inerte.

La présente invention concerne donc aussi ces charges catalytiques.

Ces chalcogénures peuvent être préparés selon un procédé décrit dans J. Solid State Chem. 3 515-519 (1971).

Les différents constituants sous forme d'élément ou de sulfure, sont mélangés intimement dans un mortier en agathe puis, la poudre est pastillée, introduite dans un tube de silice, scellé sous vide dynamique ($10^{-2}$ mmHg, Argon). Le chalcogénure est obtenu après chauffage à environ 750°C. Pour avoir un produit pur il est souvent utile de procéder à plusieurs cycles : broyage - pastillage - recuit. Toutes ces opérations sont faites sous atmosphère contrôlée, dans une boite à gants sous Argon.

Ce procédé ne permet de préparer que de faibles quantités. La demanderesse a trouvé un procédé utilisable à l'échelle industrielle et qui permet aussi de préparer toute une famille de chalcogénures.

L'invention est donc un procédé de préparation de chalcogénures de formule $M_x$ $Mo_6$ $S_8$ dans laquelle x est un nombre quelconque entre 0 et 4 et M désigne un métal, caractérisé en ce qu'on effectue une réduction par l'hydrogène d'un mélange de $MoS_2$ et du métal M ou de leurs précurseurs. Le métal M peut être par exemple un alcalin, un alcalino-terreux, un élément de la colonne 3 b du tableau périodique des éléments, un lanthanide, un actinide, le plomb, l'étain, le magnésium, le zinc, le manganèse, le cadmium, le cuivre, le nickel, le fer ou le cobalt.

On ne sortirait pas du cadre de l'invention en utilisant $MoS_2$ et un précurseur du métal M, le métal M et un précurseur de $MoS_2$, un mélange de $MoS_2$, d'un précurseur de $MoS_2$ et un métal M ou toute combinaison. La réduction par l'hydrogène peut s'effectuer par simple mise en contact de l'hydrogène avec le mélange $MoS_2$,

M.

On peut opérer à toute pression d'hydrogène, la température est comprise entre 450 et 1100°C, les précurseurs de $MoS_2$ et M sont des produits qui, dans ces conditions d'hydrogénation, donnent $MoS_2$ et M. Le précurseur de $MoS_2$ peut être par exemple :

$$(NH_4)_2Mo_3S_{13} \text{ ou } (NH_4)_2MoS_4$$

Le précurseur du métal M peut être un sel tel qu'un chlorure, un sulfure, un nitrate, un sulfate, ou un acétate. Les proportions de $MoS_2$ et M dans le mélange avant l'hydrogénation sont ajustées en fonction du chalcogénure recherché et des réactivités particulières de M, ou de son précurseur. Il peut exister aussi des précurseurs mixtes, par exemple $Cu(NH_4)MoS_4$. Selon les chalcogénures qu'on veut fabriquer, ils peuvent s'utiliser seuls ou avec d'autres précurseurs du métal ou du $MoS_2$.

Il n'est pas nécessaire d'isoler $MoS_2$ ou M avant de préparer le chalcogénure, c'est-à-dire qu'on fabrique in situ les réactifs $MoS_2$ et M, puis que la réaction continue vers le chalcogénure.

Les chalcogénures $M_xMo_6S_8$ ont la même structure que les chalcogénures $Cu_xMo_6Ch_8$ qu'on a cités plus haut, c'est-à-dire qu'on y retrouve un motif $Mo_6S_8$ qui crée un réseau hôte de structure rigide. Ces chalcogénures cristallisent dans le système hexagonal rhomboédrique.

L'invention concerne aussi un procédé de préparation de chalcogénures de formule $M_xMo_6S_8$ dans laquelle x est un nombre quelconque entre 0 et 4 et M désigne un métal, caractérisé en ce qu'on effectue une insertion du métal M dans $Mo_6S_8$ existant en présence d'un courant de gaz.

On peut utiliser le métal en l'état ou l'un de ses sels. Il suffit de mélanger le métal et $Mo_6S_8$ de préférence chacun étant dans un état divisé et de chauffer pendant qu'on balaye le mélange réactionnel avec un gaz. Il est avantageux d'utiliser l'azote. La température peut se situer entre 150 et 400°C.

La présente invention concerne aussi un chalcogénure de formule $M_xMo_6S_8$ dans laquelle x est un nombre quelconque entre 0 et 4 et M désigne un métal caractérisé en ce qu'il est déposé sur un support poreux.

Ces chalcogènures sont ceux dont on vient de citer un procédé de préparation. Le support poreux peut être de l'alumine, de la silice, des silico alumines, du graphite, du charbon, du $TiO_2$, du $ZrO_2$, et en règle générale tout ce qui est utilisé comme support de catalyseur. On peut citer plus particulièrement les poudres d'alumine de granulométrie entre 20 et 200 μm et de surface entre 90 et 450 $m^2/g$, et de préférence entre 30 et 90 μm et entre 250 et 400 $m^2/g$.

Les chalcogénures supportés avec M étant le cuivre sont utiles comme catalyseurs d'oxychloration des hydrocarbures.

On peut préparer ces chalcogénures supportés en imprégnant le support de précurseurs de $MoS_2$ et du métal M, puis on effectue une réduction par l'hydrogène. Les précurseurs de $MoS_2$ et de M sont ceux qu'on a cités précédemment. On peut aussi préparer le précurseur in-situ dans les pores du support, par exemple $(NH_4)_2MoS_4$ peut être préparé par réaction entre le paramolybdate $(NH_4)_6Mo_7O_{24}$-$4H_2O$ en solution dans $NH_4OH$ avec $H_2S$. Par exemple $(NH_4)_2Mo_3S_{13}$ peut être préparé par réaction de $(NH_4)_6Mo_7O_{24}$-$4H_2O$ avec un polysulfure d'ammonium. Ce dernier étant lui-même préparé par action de $H_2S$ avec une solution de $NH_4OH$ et de soufre (Chem. Ber. 112 p 778-780 - 1979).

On peut aussi préparer ces chalcogénures supportés en partant d'un produit déjà déposé sur un support poreux mais n'étant pas sous forme de chalcogénures.

On peut par exemple utiliser un catalyseur d'hydrodésulfuration déjà existant tel qu'un alliage Ni-Mo.S ou Co-Mo-S déposé sur alumine. Il suffit ensuite de procéder à la réduction par l'hydrogène.

Les exemples qui suivent ont été effectués au CNRS dans le laboratoire de chimie du solide et inorganique moléculaire à l'Université de Rennes (France), et dans les laboratoires de la Société ATOCHEM.

## EXEMPLE 1 - SYNTHESE $Cu_{2.5}Mo_6S_8$ EN TUBE SCELLE

On mélange :

$$2.5\ Cu + 4\ MoS_2 + 2\ Mo$$

On pastille les poudres. On introduit dans un tube de silice scellé sous vide dynamique ($10^{-2}$ mmHg pression). On recuit à 800°C pendant 70 heures. La poudre obtenue présente un diagramme de diffraction RX. Le produit cristallise dans le système hexagonal rhomboédrique.

Paramètre de maille dans le système hexagonal : a = 9,6 Å C = 10,2 Å (il peut rester du $MoS_2$ comme impureté).

## EXEMPLE 2 - SYNTHESE $Cu_2Mo_6S_8$ PAR REDUCTION A L'HYDROGENE

$$0{,}4107\ g\ (NH_4)_2MoS_4 + 0{,}0901\ g\ CuCl_2,\ 2H_2O$$

On mélange, on broie les poudres dans un mortier en agathe puis on met dans une nacelle de silice. Réduction dans un four horizontal à 700°C pendant 89 heures, avec un débit H2 # 1,5 ml/s identification par RX : paramètre de maille hexagonale : a = 9,60 Å c = 10,22 Å (s'il reste du $MoS_2$, la réaction n'est pas terminée, poursuivre la réduction).

$MoS_2$ identifié par diagramme de diffraction RX :

ASTM n° 6-0097

et

n° 9-312

**EXEMPLE 3 - SYNTHESE $Cu_xMo_6S_8$ / $Al_2O_3$**

(Chalcogénure déposé sur un support poreux)

**1 -** Imprégnation des précurseurs

On dissout 0,4107 g $(NH_4)_2MoS_4$ dans 6,5 ml d'ammoniaque concentrée et ajoute 12 g $Al_2O_3$. On laisse 24 heures dans un dessicateur rempli de $P_2O_5$ et sous vide. Dans un four horizontal, $H_2$ pendant 8 minutes, le temps de monter la température du four de l'ambiante à 400°C, débit H2 # 0,5 ml/s = produit A.

On dissout 0,0901 g $CuCl_2$ $2H_2O$ dans 8 ml de $NH_4OHCC$ et on ajoute le produit A. On laisse 75 heures dans un dessicateur rempli de $P_2O_5$ et sous vide. On procède à la réduction de la totalité sous H2 # 1,5 ml/s pendant 89 heures, dans un four horizontal. On prélève quelques granulés. On les mets dans un tube de silice scellé sous vide dynamique et on recuit pendant 24 heures à 900°C.

**2 -** Voies heptamolybdate 2 procédés différent:

1) Voie air

On dissout 1,5 g $(NH_4)_6Mo_7O_{24}.4H_2O$ et 1,36 g $Cu(NO_3)_2.3H_2O$ dans 5 ml d'ammoniaque concentrée. On ajoute 10 g de $Al_2O_3$ et on laisse à l'air ambiant pendant 1 h 30. On étuve à l'air à 120°C pendant 2 heures. Calcination sous air à 350°C pendant 2 heures. Calcination sous air à 500°C pendant 2 heures sur une fraction ≈ 10 granulés (masse < 1 g). Sulfuration par $H_2S$ à 400°C pendant 30 minutes. Réduction H2 # 1,5 ml/s pendant 49 heures. Identification par EXAFS : Seuil K du Mo d'un mélange $Cu_xMo_6S_8$ et $MoS_2$ la valeur de x n'est pas connue mais $x \geqq 2$.

2) Voie ($^H2$,$^N2$)

On dissout 1,5 g $(NH_4)_6Mo_7O_{24}.4H_2O$ et 1,36 g $Cu(NO_3)_2.3H_2O$ dans 5 ml de $NH_4OHCC$. On ajoute 10 g de $Al_2O_3$ en granulés et on laisse à l'air ambiant pendant 1 h 30. Balayage d'azote à 120°C pendant 3 heures. Hydrogène pensant 16 heures à 200°C puis pendant 2 heures à 240°C puis 67 heures à 245°C. Azote à 500°C pendant 24 heures.

Sur une fraction ≈ 10 granulés (≈ 500 mg). Sulfuration par $H_2S$ à 400°C pendant 30 minutes. Réduction sous $H_2$ # 1,5 ml/s pendant 49 ou 89 heures. Identification par EXAFS : Seuil K du Mo d'un mélange $Cu_xMo_6S_8$ et $MoS_2$.

Les mesures EXAFS au seuil K du Mo résultent de comparaisons entre les produits imprégnés décrits ci-dessus et des poudres cristallisées, de référence $Cu_4Mo_6S_8$ et $MoS_2$ (synthèses en tube scellé).

Nous avons réalisé les mesures sur les composés de référence car il n'existe pas de biblio sur $Cu_4Mo_6S_8$ en EXAFS au seuil K du Mo. Il existe des références biblio pour $MoS_2$.

**EXEMPLE 4**

On effectue les essais d'oxychloration dans l'appareillage suivant. Le réacteur est un tube de verre vertical chargé de catalyseur. Il est alimenté en gaz par le bas permettant sa fluidisation. Des thermocouples placés dans une gaine de verre au milieu du réacteur suivent l'évolution de la température à la fois dans le temps et suivant la hauteur dans le lit de catalyseur. A la sortie du réacteur, les gaz passent sur une colonne d'abattage qui, par lavage à l'eau distillée, récupère tout le HCl qui n'a pas réagi. Ce dernier se retrouve dans une ampoule à décanter. Le reste du gaz traverse ensuite successivement un réfrigérant à eau, un réfrigérant à saumure (t = -5°C) où se condensent les solvants récupérés dans une autre ampoule à décanter, puis des ampoules de prélèvement pour les dosages des gaz restant sont balayées avant d'atteindre un compteur volumétrique dont

la mesure donne le débit gazeux en sortie. Le relevé de température, à ce niveau, sert à évaluer la correction nécessaire pour revenir aux conditions TPN (température et pression normales). La pression atmosphérique est lue sur un manomètre à Mercure.

Il y a donc trois types de prélèvements différents qui conduisent aux résultats après analyse :

- une solution aqueuse de HCl
- des solvants condensés, parmi lesquels du D 12
- des gaz de sortie : CO, $CO_2$, D12, et les réactifs qui n'ont pas réagi : $C_2H_4$, air.

On compare a) un catalyseur conventionnel constitué d'alumine de surface 357 $m^2/g$, diamètre moyen 53 µm, volume poreux 33 $cm^3$/100 g imprégnée de chlorure de cuivre et d'une substance inerte qui est de la silice de diamètre moyen 50 µm et compris entre 20 et 300 µm ; avec b) un catalyseur formé d'un chalcogénure $Cu_{2.5}Mo_6S_8$, d'alumine et d'une substance inerte (silice). L'alumine et la silice ont les mêmes caractéristiques qu'en a sauf l'absence de cuivre.

Les résultats figurent dans le tableau 1.

**TABLEAU 1**

Conditions opératoires :

| | | | |
|---|---|---|---|
| - alimentation : | $C_2H_4$ | 4.4 | Nl/h |
| | AIR | 16.2 | Nl/h |
| | HCl | 8.6 | Nl/h |
| - réacteur | diamètre | 20 | mm |

| | $Cu_{2.5}Mo_6S_8$ 4.25 g<br>$Al_2O_3$ 6.00 g<br>$SiO_2$ 22.60 g | alumine avec cuivre 9.70 g<br>$SiO_2$ 23.60 g<br>catalyseur conventionnel |
|---|---|---|
| Quantité Cu (g) | 0.68 | 0.58 |
| T (°C) | 230 | 240 |
| Taux de conversion de $C_2H_4$ | 78.5 | 72.3 |
| Sélectivité en D 12 | 99.2 | 99.1 |

La charge catalytique de l'invention prouve une meilleure conversion et une meilleure sélectivité (la sélectivité exprime la fraction de $C_2H_4$ convertie qui a été transformée en D 12).

## EXEMPLE 5 - OXYCHLORATION

Débits utilisés à l'entrée du réacteur

air : 12.2 Nl/h $C_2H_4$ : 4.4 Nl/h HCl : 8.6 Nl/h pour 1.2 g de cuivre pour 59 g de charge catalytique testée au total (Cu inclus), sauf test 2.

Définitions

$X_G$ taux de conversion de $C_2H_4$
$Y_G$ taux de conversion de HCl
$Z_G$ taux de conversion de $O_2$

Liste des tests

1 $Al_2O_3$ : 12.0 g
$SiO_2$ : 39.6 g
$Cu_{2.5}Mo_6S_8$ : 7.4 g

2 $Cu_{2.5}Mo_6S_8$ : 63.9 g

4 $Al_2O_3$ : 51.6 g
$Cu_{2.5}Mo_6S_8$ : 7.4 g

6 Idem à 4

15 $Al_2O_3$ : 29.0 g
$CuCl_2/Al_2O_3$ : 30.0 g
(4 % Cu)

16 $Al_2O_3$ : 55.8 g
$CuCl_22H_2O$ : 3.2 g

L'alumine imprégnée ou non avec du cuivre ($Al_2O_3$) utilisée dans les tests ci-dessus est identique à celle de l'exemple 4. Il en est de même pour la silice.

TABLEAU 2

| test | durée | Temp. (°C) | temps de séjour(s) | XG % | YG % | ZG % | rendement CO+CO2 | rendement C1+C2 | sélectivité D12 |
|---|---|---|---|---|---|---|---|---|---|
| 1 | 4h25 | 249 | 4.5 | 88.3 | 64.9 | 78.6 | 0.7 | 1.7 | 97.3 |
| 2 | 0h15 | 264 | 4.8 | 3.1 | 2.1 | 17.1 | 1.7 | 0.3 | 36.0 |
| 4 | 4h00 | 216 | 5.9 | 32.2 | 42.8 | 26.1 | 0.8 | 3.1 | 87.9 |
| 6 | 4h00 | 249 | 5.6 | 54.4 | 46.5 | 64.2 | 8.5 | 5.0 | 75.2 |
| 16 | 4h00 | 241 | 5.8 | 26.6 | 16.9 | 11.3 | 0.4 | 13.5 | 47.3 |

C1 et C2 désignent les produits chlorés en C1 et C2 autres que le D 12.

**EXEMPLE 6**

Synthèse $Cu_xMo_6S_8$ par insertion dans $Mo_6S_8$ sous azote. La synthèse de $Mo_6S_8$ est décrite dans Rev. Chim. Min. 21, 509 (1984).

Nous avons inséré du cuivre dans $Mo_6S_8$ sous azote à une température supérieure ou égale à 200°C. Le cuivre de départ peut être métallique, ou sous forme de chlorures de cuivre.

Les réactifs sont dans les proportions stoéchiométriques Mo:Cu = 6:x.

**EXEMPLE 7**

Synthèse $Cu_xMo_6S_8/Al_2O_3$ à partir de catalyseur existant type : $MoO_3$, $NiO/Al_2O_3$ (Mo : 9 %-Ni + Mo : 12 %) $Al_2O_3$ de grande surface.

Procédé

Sulfuration sur 500 mg, par $H_2/H_2S$ 15 %, à 400°C, pendant 4 heures.

On procède ensuite à la réduction sous $H_2$ à 800°C, pendant 66 heures (débit $H_2$ différent ou égal de 1 ml/s). Le produit est identifié par son diagramme de diffraction RX après recuit en tube scellé sous vide à 900°C pendant 24 heures.

## Revendications

**1.** Procédé d'oxychloration d'un hydrocarbure pour former un hydrocarbure chloré dans lequel l'hydrocarbure, un gaz contenant de l'oxygène et de l'acide chlorhydrique gazeux passent sur une charge comprenant un chalcogénure ternaire de molybdène contenant du cuivre et de formule $Cu_xMo_6Ch_8$ dans laquelle x est un nombre quelconque entre 0 et 4 et Ch représente le soufre, le selenium ou le tellure.

**2.** Procédé selon la revendication 1, caractérisé en ce qu'on ajoute au chalcogénure un catalyseur d'oxychloration et éventuellement une substance catalytiquement et chimiquement inerte.

**3.** Procédé selon la revendication 2, caractérisé en ce que le catalyseur d'oxychloration est de l'alumine imprégnée d'un sel de cuivre et que la substance inerte est de la silice.

**4.** Procédé selon la revendication 1, caractérisé en ce qu'on ajoute au chalcogénure un support de catalyseur et éventuellement une substance catalytiquement et chimiquement inerte.

**5.** Procédé selon la revendication 4, caractérisé en ce que le support de catalyseur est de l'alumine et que la substance inerte est de la silice.

**6.** Charge catalytique, caractérisée en ce qu'elle comprend un mélange (i) de chalcogénures ternaires de molybdène de formule $Cu_xMo_6Ch_8$ dans laquelle x est un nombre quelconque entre 0 et 4 et Ch représente le soufre, le selenium ou le tellure et (ii) de catalyseurs d'oxychloration et eventuellement une charge catalytiquement et chimiquement inerte.

**7.** Charge catalytique caractérisée en ce qu'elle comprend un mélange (i) de chalcogénures ternaires de molylbdène de formule $Cu_xMo_6Ch_8$ dans laquelle x est un nombre quelconque entre 0 et 4 et Ch représente le soufre, le selenium ou le tellure et (ii) de supports de catalyseurs d'oxychloration et éventuellement une charge catalytiquement et chimiquement inerte.

**8.** Procédé de préparation de chalcogénures de formule $M_xMo_6S_8$ dans laquelle x est un nombre quelconque entre 0 et 4 et M désigne un métal, caractérisé en ce qu'on effectue une réduction par l'hydrogène d'un mélange de $MoS_2$ et de métal M ou de leurs précurseurs.

**9.** Chalcogénures de formule $M_xMo_6S_8$ dans laquelle x est un nombre quelconque entre 0 et 4 et M désigne un métal, caractérisé en ce qu'il est déposé sur un support poreux.

**10.** Procédé de préparation des chalcogénures selon la revendication 9, caractérisé en ce qu'on effectue une réduction par l'hydrogène du support poreux imprégné des $MoS_2$ et M ou de leurs précurseurs.

**11.** Procédé selon la revendication 10, caractérisé en ce que la préparation du support imprégné des précurseurs est effectuée à partir d'alliages Ni-Mo-S ou Co-Mo-S déposés sur un support poreux, l'ensemble pouvant être déjà un catalyseur d'hydrodésulfuration.

**12.** Procédé de préparation de chalcogénures de formule $M_xMo_6S_8$ dans laquelle x est un nombre quelconque entre 0 et 4 et M désigne un métal, caractérisé en ce qu'on effectue une insertion du métal M dans $Mo_6S_8$ existant en présence d'un courant de gaz.

Office européen des brevets

# RAPPORT DE RECHERCHE EUROPEENNE

Numero de la demande

EP 91 40 2533

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| E,X | EP-A-0 335 987 (MATSUSHITA ELECTRIC INDUSTRIAL)<br>* page 11; revendications 1-4 *<br>* page 3; exemples 1-7 * | 8,12 | B01J27/051<br>B01J27/51<br>C07C17/152<br>C01G39/00 |
| Y | | 9,10 | |
| Y | US-A-3 796 795 (P. URBAN)<br>* colonne 14; revendication 1 * | 9,10 | |
| Y | US-A-4 560 470 (K. MC CARTY)<br>* colonne 11; revendication 1 *<br>* colonne 5, ligne 47 - ligne 55 * | 1 | |
| Y | DE-A-1 817 281 (SHOWA DENKO KABUSHIKI KAISHA)<br>* page 16; revendications 1,5 * | 1 | |
| A | FR-A-1 440 450 (FARBWERKE HOECHST) | | |

| DOMAINES TECHNIQUES RECHERCHES (Int. Cl.5 ) |
|---|
| B01J |
| C07C |

Le présent rapport a été établi pour toutes les revendications

| Lieu de la recherche | Date d'achèvement de la recherche | Examinateur |
|---|---|---|
| LA HAYE | 11 DECEMBRE 1991 | THION M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)